# EUROPEAN PATENT APPLICATION

(11) **EP 3 769 679 A1**
(43) Date of publication of application: **27.01.2021**
(21) Application number: 19772280.4
(22) Date of filing: 08.02.2019
(51) Int. Cl.: A61B 5/1455, A61B 5/02

(54) **BIOLOGICAL INFORMATION MEASUREMENT DEVICE**

(30) Priority: 20.03.2018 JP 2018052516
(71) Applicant: Dynamic Brain Lab, LLC., Tokyo 162-0842 (JP)
(72) Inventor: VALENZI Stefano, Tokyo 135-0044 (JP); JURICA Peter, Wako-shi Saitama 351-0104 (JP)
(74) Representative: Cabinet Nuss
(86) International application number: PCT/JP2019/004659
(87) International publication number: WO 2019/181268

(57) **Abstract**

A biological information measurement device includes: a sensor module (20) which includes N light-emitting elements (21) (N is an integer greater than or equal to 3) which emit light having different wavelengths and N-1 light-receiving elements (22) which receive the light having different wavelengths emitted from the light-emitting elements (21) and reflected by an organism (10) and which output a signal of an intensity corresponding to an amount of light to be received, and a signal processing unit which performs signal processing on an output signal from the light-receiving elements (22) and which generates biological information, wherein the signal processing unit includes a microcontroller which controls an amount of light to be emitted of each of the light-emitting elements (21) on the basis of an intensity of an output signal from each of the light-receiving elements (22).

## Description

### [Technical Field]

The present invention relates to a biological information measurement device. Particularly, the present invention relates to an optical biological information measurement device which measures multiple pieces of biological information.

### [Background Art]

In the related art, as an optical biological information measurement device, pulse rate monitors, pulse oximeters, and the like are known. Pulse oximeters non-invasively measure the oxygen saturation in the blood of an organism. The principle is to measure the oxygen saturation in the blood of an organism by emitting light having different wavelengths to the organism using light-emitting elements and detecting the absorbance of each light using a difference in absorbance of light by oxy-hemoglobin and reduced hemoglobin for light having a predetermined wavelength (for example, refer to Patent Literature 1).

### [Citation List]

### [Patent Literature]

### [Patent Literature 1]

Japanese Patent Laid-Open No. H06-098881

### [Summary of Invention]

### [Technical Problem]

Incidentally, although the pulse oximeter described in Patent Literature 1 includes two light-emitting elements and one light-receiving element, for example, it has been difficult to obtain biological information including multiple blood components such as measuring an oxygen saturation in blood and a glucose concentration in blood. In order to obtain multiple pieces of biological information, a separate light-emitting elements and a light-receiving element are required.

However, there is a problem that simply increasing the numbers of light-emitting elements and light-receiving elements requires a complicated circuit to generate biological information and does not achieve a decrease in size and saving of power in a device.

An object of the present invention is to provide a biological information measurement device in which a decrease in size and saving of power in a device are achieved through a simple configuration and multiple pieces of biological information can be obtained.

In order to achieve the above object, a biological information measurement device of an aspect of the present invention includes: a sensor module which includes N light-emitting elements (N is an integer greater than or equal to 3) which emit light having different wavelengths and N-1 light-receiving elements which receive light having different wavelengths emitted from the light-emitting elements and reflected by an organism and which output a signal of an intensity corresponding to an amount of light which is received; and a signal processing unit which performs signal processing on an output signal from the light-receiving elements and which generates biological information, wherein the signal processing unit includes a microcontroller which controls an amount of light emitted by each of the light-emitting elements on the basis of an intensity of an output signal from each of the light-receiving elements.

### [Advantageous Effects of Invention]

According to the present invention, it is possible to provide a biological information measurement device in which a decrease in size and saving of power in the device are achieved through a simple configuration and multiple pieces of biological information can be obtained.

### [Brief Description of Drawings]

Fig. 1 is a diagram illustrating an example of a configuration of a sensor module in a biological information measurement device according to a first embodiment of the present invention.
Fig. 2 is a schematic plan view illustrating an example of an arrangement of light-emitting elements and light-receiving elements of a sensor module in the biological information measurement device according to the first embodiment of the present invention.
Fig. 3 is a schematic plan view illustrating an example of the arrangement of light-emitting elements and light-receiving elements of the sensor module in the biological information measurement device according to the first embodiment of the present invention.
Fig. 4 is a block diagram illustrating a functional configuration of the biological information measurement device according to the first embodiment of the present invention.
Fig. 5 is a block diagram illustrating a functional configuration of a biological information measurement device according to a second embodiment of the present invention.
Fig. 6 is a diagram illustrating an external configuration as an example of the biological information measurement device according to the second embodiment of the present invention.
Fig. 7 is a diagram illustrating an external configuration as an example of the biological information measurement device according to a third embodiment of the present invention.
Fig. 8 is a diagram illustrating an external configuration as an example of a biological information measurement device according to a fourth embodiment of the present invention.
Fig. 9 is a schematic diagram of the biological information detection device according to the third embodiment of the present invention attached to an external device.
Fig. 10 is a schematic diagram illustrating a first band section configured to install and fix a biological information measurement device of the present invention on biological tissues.
Fig. 11 is a schematic diagram illustrating a second band section configured to install and fix a biological information measurement device of the present invention on biological tissues.

### [Description of Embodiments]

Various embodiments of a biological information measurement device according to the present invention will be described below with reference to the drawings.

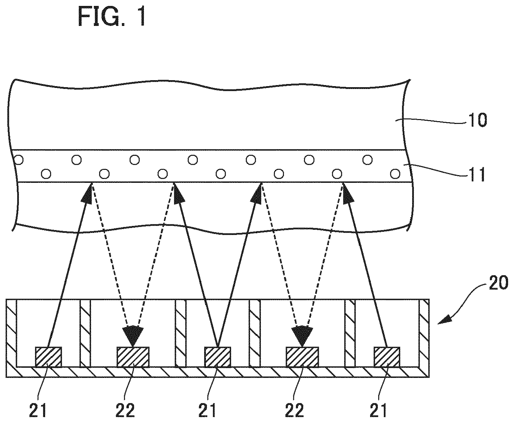

Fig. 1 is a diagram illustrating an example of a configuration of a sensor module in a biological information measurement device according to a first embodiment of the present invention. A sensor module 20 of the present invention includes three light-emitting elements 21 and two light-receiving elements 22. The sensor module 20 of the present invention is characterized in that one light-emitting element 21 is omitted as compared with the sensor module in the related art as described in Patent Literature 1.

A biological information measurement device 100 including the sensor module 20 illustrated in Fig. 1 emits light having different wavelengths from the three light-emitting elements 21 to an organism 10, receives the reflected light by the two common light-receiving elements 22, and outputs a signal of an intensity according to an amount of light received. Thus, it is possible to obtain biological information including that of multiple blood components so that a glucose concentration in blood is also measured while realizing the absorption of signal fluctuations due to movement of the body of a person on which measurement is performed and the acquisition of an oxygen saturation in blood that is a characteristic of a pulse oximeter. Furthermore, it is possible to measure the same component for the organism and calculate an average value thereof, and if one light-receiving element is operating normally, it is possible to obtain required biological information even if the other light-receiving element has an abnormality, whereby it is possible to improve the reliability of measurement values. With a simple configuration in which one light-emitting element 21 is omitted as compared with the device in the related art, an installation space required for the light-emitting element 21 in the related art, a voltage loss due to a drive circuit, and the like are eliminated and it is possible to realize a decrease in size and saving of power in the device.

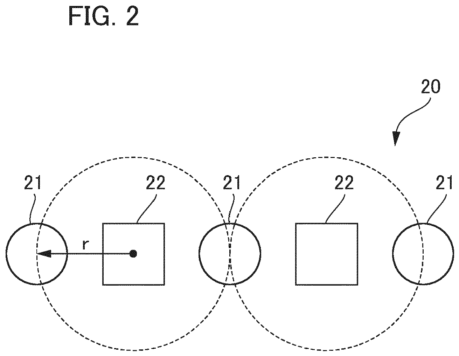

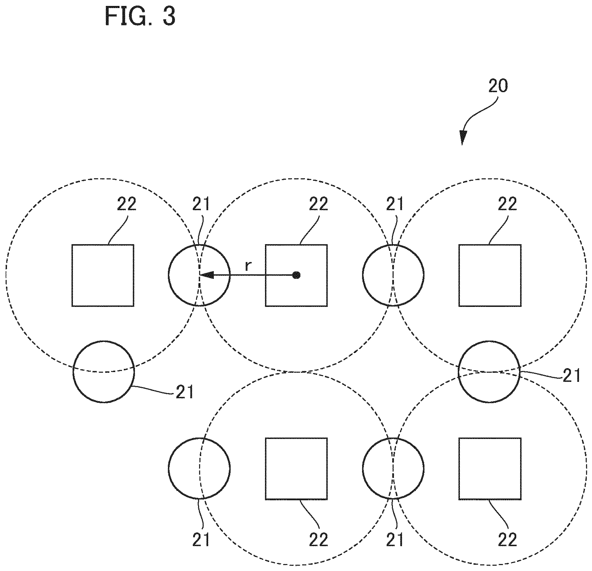

Figs. 2 and 3 are schematic plan views illustrating an example of an arrangement of light-emitting elements and light-receiving elements of a sensor module in the biological information measurement device of the present invention. The sensor module 20 includes N light-emitting elements 21 and N-1 light-receiving elements 22. Fig. 2 illustrates an example of an arrangement when N is 3 and Fig. 3 illustrates an example of an arrangement when N is 5.

A first light-emitting element 21 and a second light-emitting element 21 are arranged on a circular circumference with a radius r indicated by an alternate long and short dash line centering on a first light-receiving element 22 and the second light-emitting element 21 and a third light-emitting element 21 are arranged on a circular circumference with a radius r indicated by an alternate long and short dash line centering on a second light-receiving element 22. The radius r indicates an interval between the center of either of two light-emitting elements 21 and a common light-receiving element 22. In order to realize a decrease in size for the sensor module 20, the radius r is preferably within 1 mm. As shown in Fig. 2, a first light-emitting element, a second light-emitting element, a third light-emitting element, a first light-receiving element, and a second light-receiving element may be arranged on the same straight line.

Also, although the light-emitting elements 21 and the light-receiving elements 22 have an arrangement of being arranged in two rows in the example illustrated in Fig. 3, this is not always necessary and they may be arranged, for example, in one row or three rows. In brief, in order to realize decrease in size of the device, the light-emitting elements 21 simply need to be provided around the light-receiving elements so that distances between the light-emitting elements 21 and a common light-receiving element 22 are the same.

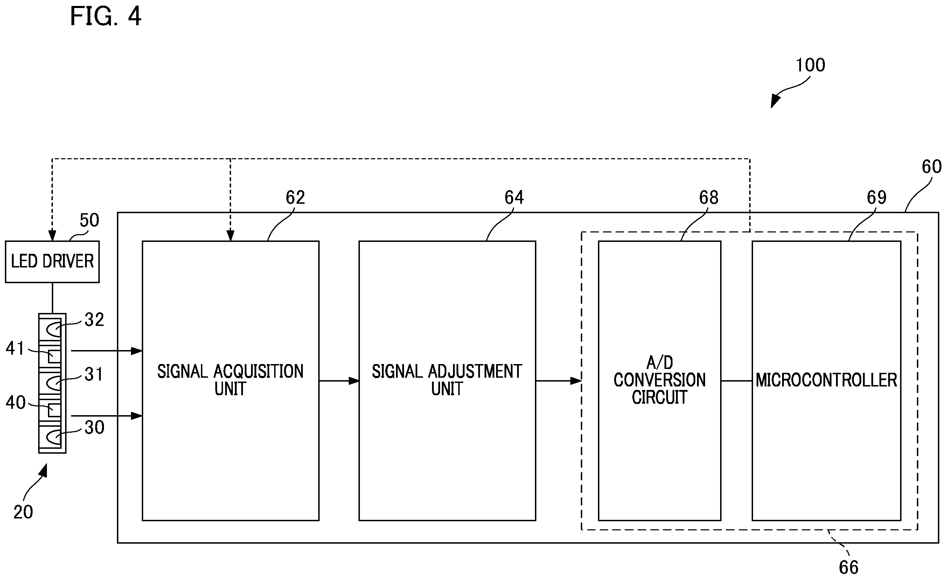

Fig. 4 is a block diagram illustrating a functional configuration of the biological information measurement device according to the first embodiment of the present invention. The biological information measurement device 100 according to this embodiment includes the sensor module 20, a light emitting diode (LED) driver 50 connected to the sensor module, and a signal processing unit 60.

The sensor module will be described in detail. Each of the light-emitting elements 21 constituting the sensor module 20 includes a red LED 30 which emits red visible light, an infrared LED 31 which emits infrared light, and a near-infrared LED 32 which emits near-infrared light. For example, the red LED 30 has a peak wavelength or a center wavelength near 660 nm, the infrared LED 31 has a peak wavelength or a center wavelength near 940 nm, and the near-infrared LED has a peak wavelength or a center wavelength near 1550 nm.

Each of the light-receiving elements 22 constituting the sensor module 20 includes a photodiode 40 which receives light having a wavelength in a red visible light region to an infrared light region and a photodiode 41 which receives light having a wavelength in an infrared light region to a near-infrared light region. Preferably, a wavelength range received by the photodiode 40 is 600 to 1000 nm and a wavelength range received by the photodiode 41 is 900 to 1600 nm.

The LED driver 50 is a drive circuit configured to drive the LEDs 30, 31, and 32 of the sensor module 20. The LED driver 50 controls amounts of light emitted by the LEDs 30, 31, and 32 under the control of a microcontroller 69 of the signal processing unit 60. Furthermore, the LED driver 50 controls a light emission cycle of each of the LEDs 30, 31, and 32 in which the respective LEDs are turned on or off under the control of the microcontroller 69.

The signal processing unit 60 includes a signal acquisition unit 62, a signal adjustment unit 64, and a signal control unit 66. The signal acquisition unit 62 includes an amplification circuit and a sample and hold circuit. The signal adjustment unit 64 includes a low pass filter and a high pass filter configured to remove noise components. The signal control unit 66 includes analog/digital (A/D) conversion circuit 68 and the microcontroller 69. The microcontroller 69 computes and calculates biological information and controls operations of units which constitute the biological information measurement device 100.

The red LED 30, the infrared LED 31, and the near-infrared LED 32 of the sensor module 20 selectively emit light using the LED driver 50 under the control of the microcontroller 69 and emit light having different wavelengths to the organism 10. Light reflected by the organism 10 is received by the photodiodes 40 and 41 and a signal corresponding to an amount of light received is output to the signal acquisition unit 62. Under the control of the microcontroller 69, the signal acquisition unit 62 distributes signals output from the photodiode 40 and the photodiode 41 to the amplification circuit and outputs the signals to the signal adjustment unit 64. At this time, the sample and hold circuit of the signal acquisition unit 62 holds signals output from the photodiodes 40 and 41 for a certain period under the control of the microcontroller 69. The low pass filter of the signal adjustment unit 64 performs low pass filtration on a signal output from the signal acquisition unit 62 at a predetermined cutoff frequency and outputs the signal to the high pass filter. The high pass filter performs high pass filtration on a signal output from the low pass filter at a predetermined cutoff frequency and outputs the signal to the signal control unit 66. Since the signal output from the high pass filter may be a weak signal which is transmitted or digitized in some cases, the signal adjustment unit 64 may further include an amplification circuit configured to amplify the signal. The A/D conversion circuit 68 of the signal control unit 66 converts a signal output from the signal adjustment unit 64 from an analog signal into a digital signal and outputs the digital signal. The microcontroller 69 performs signal processing such as waveform shaping on the basis of a digital signal output from the A/D conversion circuit 68 and generates biological information as a measurement result. The biological information to be generated includes one or more of a pulse rate, an oxygen concentration in blood, and a glucose concentration in blood. The generated biological information and signal can be stored in a memory of the microcontroller 69.

Since the biological information measurement device 100 of the present invention deals with various types of measurement of the organism 10, the microcontroller 69 can control amounts of light emitted by the LEDs 30, 31, and 32 in the LED driver 50, signal amplification in the signal acquisition unit 62, and the like in parallel on the basis of signals output from the photodiodes 40 and 41. For example, when biological tissues having a thick skin thickness are measured, relatively small amounts of light can be made to be incident on the photodiodes 40 and 41. Thus, a larger amount of light is required for achieving detection of light. In this case, the microcontroller 69 can perform control, for example, so that amounts of light emitted by the LEDs 30, 31, and 32 are increased via the LED driver 50 or directly and signal amplification of the signal acquisition unit 62 is increased. With this configuration, it is possible to provide a biological information measurement device in which a decrease in size and saving of power in the device can be realized through a simple configuration and in which multiple pieces of biological information can be obtained.

The various processes described above may be performed not only in time series in accordance with the description but also in parallel or individually in accordance with the processing capacity of the device which performs the process or necessity. Furthermore, although the embodiment in which information of the organism 10 is calculated using the microcontroller 69 has been described, biological information may be calculated through processing of a microcontroller or the like of an external device. In this case, the biological information measurement device 100 includes a communication unit which can communicate with an external device.

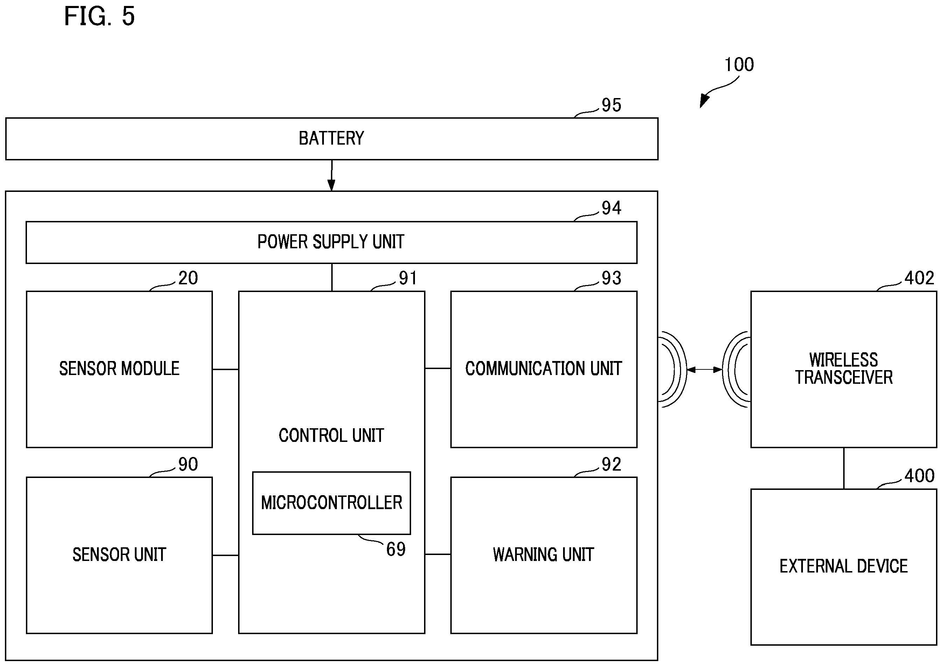

Fig. 5 is a block diagram illustrating a functional configuration of a biological information measurement device according to a second embodiment of the present invention. A biological information measurement device 100 according to the second embodiment further includes a sensor unit 90, a control unit 91, a warning unit 92, a communication unit 93, and a power supply unit 94, in addition to the biological information measurement device 100 according to the first embodiment described above.

The sensor unit 90 includes a temperature sensor, a humidity sensor, an acceleration sensor, a pressure sensor, a gyro sensor, a galvanic skin response (GSR) sensor, and the like. Various sensors constituting the sensor unit are connected to the control unit 91 and output the detected signals to the control unit 91.

The temperature sensor has a function of detecting an organism temperature of a subject, an external temperature, and the like. The humidity sensor has a function of detecting an external humidity and the like. The acceleration sensor and the gyro sensor have a function of detecting a body movement associated with a subject's body. The biological information measurement device 100 in this embodiment may be configured to include both an acceleration sensor and a gyro sensor and use the detection results of both of the sensors together or may include only one of them. The pressure sensor has a function of detecting an external pressure such as an atmospheric pressure. The pressure sensor also has a function of detecting a pressure applied to the organism 10 and the biological information measurement device 100. A GSR sensor 86 has a function of detecting a GSR (or galvanic skin response) value and the like of a subject.

The control unit 91 also functions as the signal processing unit 60 of the biological information measurement device in the first embodiment and is connected to the sensor module 20 and various sensors of the sensor unit 90 described above. The control unit 91 includes a microcontroller 69 and controls the overall operation of the biological information measurement device 100. The control unit 91 also has a function of storing a control program to be executed, processing results, various data, and the like. The control unit 91 controls various sensors of the sensor module 20 and the sensor unit 90 and performs acquisition, analysis, and output operations on measurement data of biological information. This measurement data may be transmitted to an external device 400 via the communication unit 93 each time signals detected by the various sensors are acquired or may be temporarily stored in the control unit 91 and periodically transmitted. Furthermore, when the acquired measurement data is transmitted to the external device 400, the data analysis of the measurement data described above may be performed using the external device 400.

Since the signals output from the various sensors to the control unit 91 are analog signals, the control unit 91 has an AD conversion function of converting an analog signal to be input into a digital signal. Here, when the analog signal to be input is directly transmitted to the external device 400, the AD conversion function may be not provided.

Examples of the biological information to be obtained include sweating, a heartbeat, pulse waves, a skin temperature, a galvanic skin response, a skin resistance value, an oxygen saturation in blood, a glucose concentration in blood, and the like. In addition, the biological information may be appropriately selected in accordance with the purpose of utilization of the biological information measurement device 100.

The warning unit 92 includes a light output unit which outputs predetermined light or a sound output unit which outputs predetermined sound. The warning unit 92 also has a function of emitting predetermined light and sound to a person on which measurement is being performed or the like on the basis of the detection that the acquired biological information is less than or equal to a predetermined threshold value. Furthermore, when the measurement of biological information is stopped and measurement on the person on which measurement is being performed has not restarted after a certain period of time, the warning unit 92 may emit light or sound to prompt restarting of measurement on the person on which measurement is being performed.

The communication unit 93 has a function of transmitting the measurement data which has been subjected to signal processing using the control unit 91 in a wireless or wired manner to the external device 400. Furthermore, the communication unit 93 has a function of transmitting a signal used for giving a notification of abnormality to the external device 400 on the basis of the detection that the acquired biological information is less than or equal to a predetermined threshold value. In this embodiment, for example, the measurement data is transmitted to the external device 400 including a wireless transceiver 402 through a method such as infrared communication or Bluetooth (registered trademark).

Also, the communication unit 93 may transmit the measurement data to an external information device through a cable. In this case, since electric power is supplied using a power supply line of this cable, electric power is supplied at the same time as during the time of the external device 400 being connected via a cable. The cable may be a USB cable.

The power supply unit 94 includes a connection terminal used for connecting to an attachable/detachable external battery 95 or an external device 400 capable of supplying electric power, a power supply circuit, and a charging circuit. This connection terminal may be a USB terminal.

The external device 400 includes the wireless transceiver 402. For example, a mobile terminal, a tablet terminal, or the like may be exemplified. The external device 400 may include a display unit which can display measurement data and the like received from the communication unit 93. Thus, a person on which measurement is performed using the biological information measurement device 100 can confirm his/her current or previous biological information using numerical values.

The biological information measurement device 100 and the external device 400 are not limited to the configuration of this embodiment and various modifications such as omission of some of these constituent elements and addition of other constituent elements are possible.

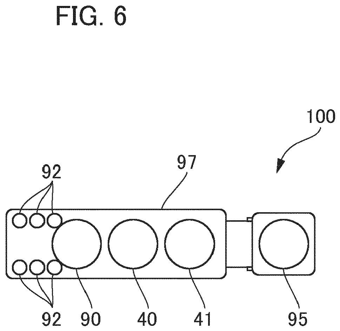

Fig. 6 is a diagram illustrating an external configuration as an example of the biological information measurement device according to the second embodiment of the present invention. Apparently, the biological information measurement device 100 according to this embodiment includes a housing 97, two photodiodes 40 and 41, a sensor unit 90, a warning unit 92 which outputs predetermined light and sound, and an attachable/detachable external battery 95. In this embodiment, the housing 97 is a substantially rectangular parallelepiped box-shaped member and includes a light transmission window. The light transmission window is formed of a member through which light is transmitted and light transmitted through the light transmission window is received by a light-receiving element of a sensor module. The inside of the housing 97 serves as an arrangement space for various sensors and the like. In this embodiment, the device is a wearable type device without a wired cable. It is desirable that a thickness of the housing 97 be thinner and the thickness thereof may be less than or equal to 3 mm.

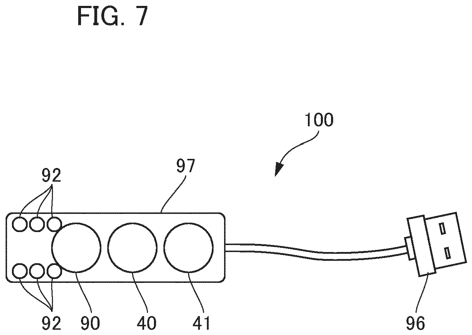

Fig. 7 is a diagram illustrating an external configuration as an example of a biological information measurement device according to a third embodiment of the present invention. Apparently, a biological information measurement device 100 according to this embodiment includes a housing 97, two photodiodes 40 and 41, a sensor unit 90, a warning unit 92 which outputs predetermined light and sound, and a cable 96. The biological information measurement device 100 according to this embodiment receives electric power supplied from an external device 400 via the cable 96. Furthermore, it is possible to transmit data to the external device 400 via the cable 96. The cable 96 may be a USB cable. In this embodiment, the device is a portable type device.

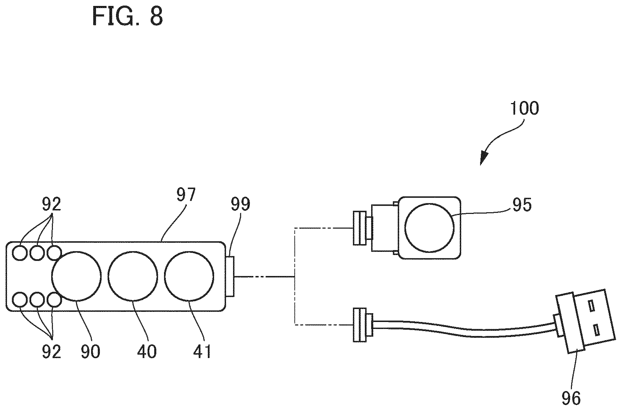

Fig. 8 is a diagram illustrating an external configuration as an example of a biological information measurement device according to a fourth embodiment of the present invention. Apparently, a biological information measurement device 100 according to this embodiment includes a housing 97, two photodiodes 40 and 41, a sensor unit 90, a warning unit 92 which outputs predetermined light and sound, and a connection terminal 99. The connection terminal 99 can be connected to any external device 400 via a battery 95 and a cable 96. The connection terminal 99 may be a USB connection terminal.

The fourth embodiment may be any device of the wearable type device illustrated in the second embodiment and the portable type device illustrated in the third embodiment. According to the fourth embodiment, it is not necessary to separately manufacture the wearable type device and the portable type device and it is possible to reduce man-hours and manufacturing costs.

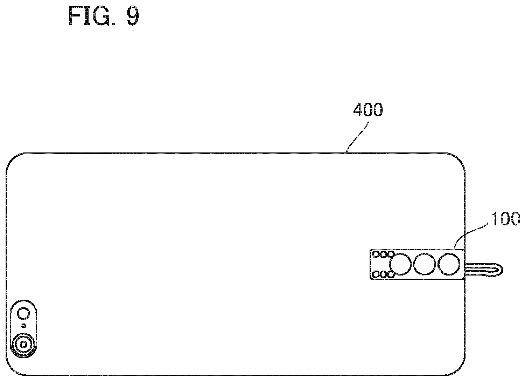

Fig. 9 is a schematic diagram of the biological information detection device according to the third embodiment of the present invention attached to an external device. The biological information measurement device 100 is connected to the external device 400 via the cable 96 and receives electric power supplied from the external device 400 or performs data communication with the external device 400. If the external device 400 includes a display unit, a configuration in which the measurement data obtained using the biological information measurement device 100 is displayed in the external device 400 is provided. Since a decrease in size of the sensor module 20 of the present invention is realized, a thickness thereof is reduced and a size and a thickness of the housing 97 of the biological information measurement device 100 according to this embodiment are relatively reduced. For this reason, as shown in Fig. 8, the biological information measurement device 100 according to this embodiment can be provided on an upper surface of the external device 400 and has excellent portability without limiting the behavior of a person to be measured.

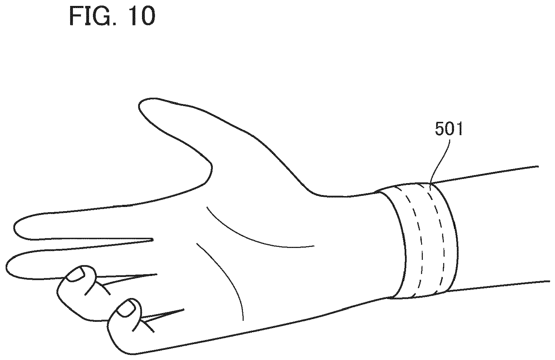

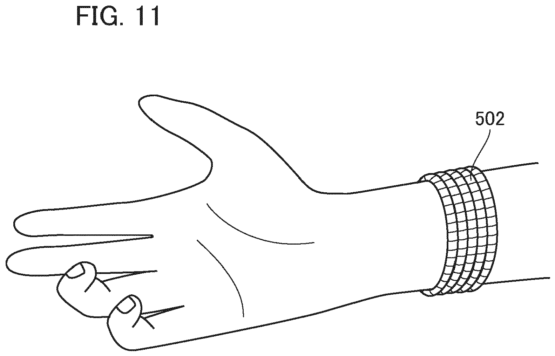

Figs. 10 and 11 are schematic diagrams illustrating band sections configured to install and fix a biological information measurement device of the present invention on biological tissues. The band sections includes a first band section 501 and a second band section 502. The first band section 501 illustrated in Fig. 10 has an internal surface so that the first band section 501 holds the biological information measurement device 100 in an attachable/detachable manner and comes into contact with biological tissues. In Fig. 10, for example, a hole portion in which the biological information measurement device 100 can be stored is provided in a dotted line portion shown in the drawing of the first band section 501. The second band section 502 illustrated in Fig. 11 is installed above the first band section 501 and includes a stretchable light-shielding member which shields light leaking between the second band section 502 and a surface coming into contact with biological tissues or light leaking directly.

Although the band type biological information measurement device 100 worn around the wrist of the organism has been described as an example in Figs. 10 and 11, the biological information measurement device 100 to which the present invention is applicable is not limited thereto. For example, it is also possible to apply to a biological information measurement device 100 such as a spectacles types in which a measurement device is stored in a frame of spectacles.

The above-described embodiments are merely examples of specific embodiments for carrying pout the present invention and the technical scope of the present invention need not be limitedly interpreted by there. That is to say, the present invention can be implemented in various forms without departing from the gist of the main features thereof.

Also, it is needless to say that the respective embodiments and the operation configurations described above can be implemented in any combination as long as they do not contradict each other.

Priority is claimed on Japanese Patent Application No. 2018-052516, filed March 20, 2018, the entire contents of which is incorporated herein by reference.

### [Reference Signs List]

10 Organism
11 Blood
20 Sensor module
21 Light-emitting element
22 Light-receiving element
30 Signal processing unit
30 Red LED
31 Infrared LED
32 Near-infrared LED
40 Photodiode
41 Photodiode
60 Signal processing unit
62 Signal acquisition unit
64 Signal adjustment unit
66 Signal control unit
68 A/D conversion circuit
69 Microcontroller
100 Biological information measurement device

## Claims

1. A biological information measurement device (100), comprising:
a sensor module (20) which includes N light-emitting elements (21) (N is an integer greater than or equal to 3) which emit light having different wavelengths and N-1 light-receiving elements (22) which receive light having different wavelengths emitted from the light-emitting elements (21) and reflected by an organism (10) and which output a signal of an intensity corresponding to an amount of light received; and
a signal processing unit (30) which performs signal processing on an output signal from the light-receiving elements (22) and which generates biological information,
wherein the signal processing unit (30) includes a microcontroller (69) which controls an amount of light emitted by each of the light-emitting elements (21) on the basis of an intensity of an output signal from each of the light-receiving elements (22).

2. The biological information measurement device (100) according to claim 1, wherein the microcontroller (69) controls amplification of the output signal from the light-receiving element (22) on the basis of the intensity of the output signal from the light-receiving element (22).

3. The biological information measurement device (100) according to claim 1 or 2, wherein the N light-emitting elements (21) include light-emitting diodes (LEDs) which emit red visible light, LEDs which emit infrared light, or LEDs which emit near-infrared light.

4. The biological information measurement device (100) according to any one of claims 1 to 3, wherein the N-1 light-receiving elements (22) includes a photodiode which generates a signal corresponding to an amount of light received of light having a wavelength from a red visible light region to an infrared light region or an infrared light region to a near-infrared light region.

5. The biological information measurement device (100) according to claim 1, wherein the sensor module (20) includes:
a first light-emitting element (21) which emits light having a first wavelength;
a second light-emitting element (21) which emits light having a second wavelength;
a third light-emitting element (21) which emits light having a third wavelength;
a first light-receiving element (22) which receives light having the first wavelength and the second wavelength emitted from the first light-emitting element (21) and the second light-emitting element (21) and reflected by an organism (10) and which outputs a signal of an intensity corresponding to an amount of light to be received; and
a second light-receiving element (22) which receives light having the second wavelength and the third wavelength emitted from the second light-emitting element (21) and the third light-emitting element (21) and reflected by an organism (10) and which outputs a signal of an intensity corresponding to an amount of light to be received,
wherein the microcontroller (69) controls at least one of amounts of light emitted by the first light-emitting element (21), the second light-emitting element (21), and the third light-emitting element (21) on the basis of intensities of the output signals from the first light-receiving element (22) and the second light-receiving element (22).

6. The biological information measurement device (100) according to claim 5, wherein the microcontroller (69) controls amplification of the output signals from the first light-receiving element (22) and the second light-receiving element (22) on the basis of the intensities of the output signals from the first light-receiving element (22) and the second light-receiving element (22).

7. The biological information measurement device (100) according to claim 5 or 6, wherein the first wavelength is a wavelength in the red visible light region, the second wavelength is a wavelength in the infrared light region, and the third wavelength is a wavelength in the near-infrared light region,
the first light-emitting element (21) is a red LED (30), the second light-emitting element is an infrared LED (31), and the third light-emitting element is a near-infrared LED (32), and
the first light-receiving element (22) is a photodiode (40) which receives light having a wavelength in the red visible light region to the infrared light region and the second light-receiving element (22) is a photodiode (41) which receives light having a wavelength in the infrared light region to the near-infrared light region.

8. The biological information measurement device (100) according to any one claims 1 to 7, wherein the biological information includes one or more of a pulse rate, an oxygen concentration in blood, and a glucose concentration in blood.

9. The biological information measurement device (100) according to any one claims 1 to 8, further comprising:
a sensor unit (90) which includes one or more of a temperature sensor configured to detect a temperature of the organism (10) or an outside temperature, a humidity sensor configured to detect external humidity, a pressure sensor configured to detect an external pressure, an acceleration sensor configured to detect movement of the organism, a gyro sensor, and a galvanic skin response (GSR: or galvanic skin reflex) sensor configured to detect a GSR of the organism (10);
a communication unit (93) which communicates with an external device (40); and
a control unit (91) which causes the communication unit (93) to transmit a detection value obtained by the sensor unit (90) to the external device (400) together with the biological information.

10. The biological information measurement device (100) according to claim 9, wherein the control unit (91) detects an abnormality by comparing the biological information with a threshold value, and
the biological information measurement device (100) includes: a warning unit (92) which outputs light or sound or which transmits a notification signal concerning the abnormality to the external device (400) via the communication unit (93) if the control unit (91) detects an abnormality.

11. The biological information measurement device (100) according to any one claims 1 to 10, comprising:
a band section configured to install the sensor module (20) on a measurement site of the organism (10),
wherein the band section includes a first band section (501) which holds the sensor module (20) and which has an opening portion through which the sensor module (20) comes into contact with the organism (10), and
a second band section (502) which is provided on an outer circumference of the first band section (501) and which shields light leaking between the organism and the sensor module (20).
